# EUROPEAN PATENT APPLICATION

(11) **EP 4 016 080 A1**
(43) Date of publication of application: **22.06.2022**
(21) Application number: 19942193.4
(22) Date of filing: 16.08.2019
(51) Int. Cl.: G01N 33/536, G01N 33/543, G01N 33/569

(54) **WHOLE NEW IMMUNOASSAY MODE FOR DETERMINING TOTAL ANTIBODY**

(71) Applicant: Shenzhen Mindray Bio-Medical Electronics Co., Ltd., Shenzhen, Guangdong 518057 (CN)
(72) Inventor: WANG, Di, Shenzhen, Guangdong 518057 (CN); HE, Jianwen, Shenzhen, Guangdong 518057 (CN); XIA, Ying, Shenzhen, Guangdong 518057 (CN)
(74) Representative: KIPA AB
(86) International application number: PCT/CN2019/100978
(87) International publication number: WO 2021/030935

(57) **Abstract**

Disclosed are a novel immunoassay format design for determining a total antibody, and a kit accordingly provided for detecting antibodies of a pathogen or pathogens of infectious diseases within a human blood sample, wherein the kit comprises: a first reagent containing at least one antigen coated on a solid phase support and an anti-human IgM antibody coated on a solid phase support; and a second reagent containing at least one labelled antigen and a labelled anti-human IgG antibody, wherein at least one antigen of the at least one antigen coated on a solid phase support and at least one antigen of the at least one labelled antigen can bind to the same IgG antibody or the same IgM antibody in the sample. The kit can overcome the disadvantages caused by detection principles while retaining the advantages of each detection principle. In addition, also provided is a new method for detecting an antibody produced after the infection of a pathogen or pathogens in a sample.

## Description

### TECHNICAL FIELD

The disclosure relates to the field of in vitro diagnosis, and specifically to a novel immunoassay format for measuring total antibodies, and particularly an immunoassay kit and a method for detecting specific antibodies.

### BACKGROUND

For in vitro diagnosis of infectious diseases, it is important to detect one or more classes of antibodies specifically against certain antigens.

Antibodies (also referred to as immunoglobulins) are produced by the immune system for defending against "foreign" substances (also referred to as antigens). In human, immunoglobulins can be divided into five different types: IgM, IgG, IgA, IgE, and IgD.

When a certain foreign antigen enters the body, the antibody firstly secreted in the immune response is IgM antibody, which binds to the antigen and activates a complement cascade. However, IgG antibody is the main component of serum immunoglobulins, which accounts for 75% of the total immunoglobulins, and is the most persistent and most important antibody in the primary immune response. The measurement of IgG and IgM antibodies against a certain pathogen can cover different stages of an infection, such as an acute infection stage, a recurrent infection stage, a chronic/persistent infection stage or a post-infection disease stage/recovered stage, therefore, is significant to the detection for the existence of an antigen specifically from a pathogen. The pathogen may be a virus or a spirochete or other microorganisms.

Therefore, in the field of in vitro diagnosis of antibodies, there is a strong need for simultaneous detections of IgG and IgM antibodies specific for a pathogen to assist in assessing disease stages.

In addition, assays such as recomLine, enzyme-linked immunosorbent assay, ELISA-plate-based chemiluminescent immunoassay, and magnetic-particle-based chemiluminescent immunoassay, etc. can be used for the detection of antibodies against pathogens of infectious diseases.

At present, the detection principles of these assays are usually based on double antigens sandwich method, indirect method or immunocapture method. In the double antigens sandwich method, a solid phase carrier and a tracer are respectively labelled with an antigen, and two variable regions of the antibody to be tested in a sample can be bound thereto. In the indirect method, a solid phase carrier is coated with an antigen, and a tracer is linked with a secondary antibody. In the immunocapture method, a solid phase carrier is coated with a secondary antibody, and a tracer is linked with a specific antigen or antigens. However, each detection method based on the principles above has certain drawbacks.

For the double antigens sandwich method, the antibody analyte may bind to the antigens on two solid phases or the antigens on two tracers during a detection process, which may result in false negative detection.

For the indirect method, a considerable number of non-specific immunoglobulins (such as, 700-1600 mg/dL of IgG and 40-230 mg/dL of IgM) are simultaneously present in the sample to be tested, which may non-specifically bind to a solid phase to varying degrees. Therefore, when a secondary antibody labelled with a tracer is used, these non-specifically bound immunoglobulins will also be recognized and bound, which results in an increase in background signal and a decrease in sensitivity. And, the indirect format is also very susceptible to heterophilic antibodies interference, consequently, causing false positive results.

For the immunocapture method, similar to the indirect method, there may be non-specific binding in a reaction system due to the presence of a secondary antibody, which will in turn affect detection results.

Therefore, in the field of in vitro diagnosis of antibodies, there is a need to overcome the drawbacks caused by the above detection principles, respectively.

### SUMMARY

In order to avoid the problems of current detection methods, the inventors have studied the methods of in vitro antibody assay and found unexpectedly a new immunoassay format that combines the principles of the double antigens sandwich method, the indirect method and the immunocapture method simultaneously, which not only largely eliminates the drawbacks caused by each assay principle illustrated above, but also synergically retains the advantages of the three methods, and thus have completed the disclosure.

In a first aspect, the disclosure provides a kit, including:
a first reagent containing at least one antigen and an anti-human IgM antibody, the antigen and the anti-human IgM antibody are coated on a solid phase support, and
a second reagent containing at least one labelled antigen and a labelled anti-human IgG antibody,
wherein the at least one antigen coated on the solid phase support and the at least one labelled antigen are capable of binding to a same IgG antibody or a same IgM antibody in the sample.

The kit of the disclosure can be used for the immunoassay of a total antibody (predominantly, IgG+IgM).

In the kit of the disclosure, the antigen used in the first reagent and the antigen used in the second reagent may form a sandwich structure (antigen-antibody-antigen) with the same IgG antibody or IgM antibody to be tested; the antigen used in the first reagent and the anti-human IgG antibody used in the second reagent may bind to the same IgG antibody to be tested; and the anti-human IgM antibody used in the first reagent and the antigen used in the second reagent may bind to the same IgM antibody to be tested.

The antibody tested by the kit of the disclosure refers to a corresponding antibody produced after the infection of a pathogen in a human body.

In an exemplary embodiment, the types of antibodies detected are IgG and IgM.

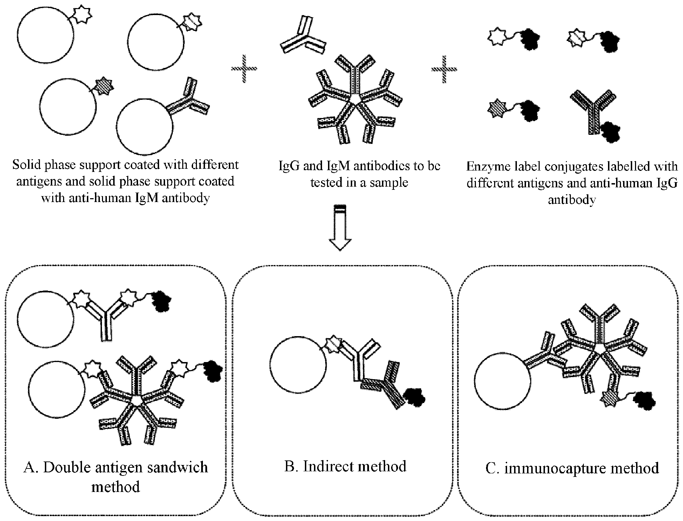

FIG. 1 exemplarily shows the reaction principles when the kit of the disclosure is used for detection. When the kit of the disclosure is used, the following reactions may occur: as shown in A in FIG. 1 (only one of the antigens is taken as an example), one or more antigens in the first reagent, the IgG antibody to be detected and one or more antigens in the second reagent can form a complex; and one or more antigens in the first reagent, the IgM antibody to be detected and one or more antigens in the second reagent can form an antigen-antibody complex. As shown in B in FIG. 1 (only one of the antigens is taken as an example), the antigen in the first reagent, the IgG antibody to be detected and the anti-human IgG antibody in the second reagent can form an antigen-antibody complex. As shown in C in FIG. 1 (only one of the antigens is taken as an example), the anti-human IgM antibody in the first reagent, the IgM antibody to be detected and the antigen in the second reagent can form an antigen-antibody complex.

In another exemplary embodiment, the anti-human IgM antibody in the first reagent may be present at a concentration of about 10 ng/mL to about 30 ng/mL, and the anti-human IgG antibody in the second reagent may be present at a concentration of about 100 ng/mL to about of 300 ng/mL. In particular, the concentration of the anti-human IgG antibody in the second reagent is approximately 10 times the concentration of the anti-human IgM antibody in the first reagent.

In an alternative kit of the disclosure, the variant includes:
a first reagent containing at least one antigen and an anti-human IgG antibody, the antigen and the anti-human IgM antibody are coated on a solid phase support, and
a second reagent containing at least one labelled antigen and a labelled anti-human IgM antibody,
wherein the at least one antigen coated on the solid phase support and the at least one labelled antigen are capable of binding to a same IgG antibody or a same IgM antibody in the sample.

When the alternative kit is used, the following reactions may be performed: one or more antigens in the first reagent, the IgG antibody to be detected and one or more antigens in the second reagent can form an antigen-antibody complex; one or more antigens in the first reagent, the IgM antibody to be detected and one or more antigens in the second reagent can form an antigen-antibody complex; the antigens in the first reagent, the IgM antibody to be detected and the anti-human IgM antibody in the second reagent can form a complex; and the anti-human IgG antibody in the first reagent the IgG antibody to be detected and the antigen in the second reagent can form an antigen-antibody complex.

In a still another exemplary embodiment, the anti-human IgG antibody in the first reagent may be present at a concentration of about 100 ng/mL to about 300 ng/mL, and the anti-human IgM antibody in the second reagent may be present at a concentration of about 10 ng/mL to about of 30 ng/mL. In particular, the concentration of the anti-human IgG antibody in the first reagent is approximately 10 times the concentration of the anti-human IgM antibody in the second reagent.

The kit of the disclosure can simultaneously detect a total antibody (predominately IgG antibody and IgM antibody) in a sample, so that the detection covers all periods of the infection of a pathogen, thereby facilitating the prevention, diagnosis, and treatment of related infectious diseases.

In addition, when a seroconversion panel is tested, a kit only based on the double antigens sandwich method or only based on the immunocapture method has the defects of low detection rate and non-obvious gradient of detected samples. However, when the kit of the disclosure is used to test the seroconversion panel, the above-mentioned defects may be overcome. In addition, when an IgM positive sample is tested, a kit only based on the indirect method or only based on the double antigens sandwich method may demonstrate low detection rate. However, when the kit of the disclosure is used to test the IgM positive sample, the above-mentioned defect may be overcome. When a negative sample is tested, a kit only based on the indirect method or only based on the immunocapture method has the defects of poor specificity and high false positive rate. However, when the kit of the disclosure is used to test the negative sample, the above-mentioned defects may be overcome.

The kit of the disclosure simultaneously involves three detection principles of the double antigens sandwich method, the indirect method and the immunocapture method. It is worth noting that, by using the kit of the disclosure, the advantages of the three principles are basically maintained during the detection process, and the defects of each principle could be overcome. It is generally considered that when different detection principles are used in combination, due to the coexistence of multiple detection systems, on the one hand, it is not excluded that the combined use may bring the advantages of each principle; however, on the other hand, the combined use may also introduce the defects of each principle. As confirmed in the following examples, when the negative sample is tested, the kit based on the principles of both the double antigens sandwich method and the indirect method shows poorer specificity and more false positive result compared with the kit only based on the double antigens sandwich method; and compared with the kit only based on the indirect method, the sensitivity of the kit based on the principles of both the double antigens sandwich method and the indirect method is improved to a certain extent but not significantly when it is used to test an IgM-positive samples. That is to say, a kit that combines the principles of both the double antigens sandwich method and the indirect method may not have more obvious advantages than a kit with a single detection principle. Compared with the kit only based on the double antigens sandwich method, the kit based on the principles of both the double antigens sandwich method and the immunocapture method has significantly improved sensitivity to an IgM positive sample, but shows poorer specificity and more false positive samples when the negative samples are tested, and the sensitivity to a seroconversion panel sample is not improved; and compared with the kit only based on the immunocapture method, the kit based on the principles of both the double antigens sandwich method and the immunocapture method shows an improved but not satisfying sensitivity to a seroconversion panel sample, moreover the specificity to negative samples is not improved. That is to say, a kit that combines the principles of both the double antigens sandwich method and the immunocapture method cannot solve the defects of a kit with a single detection principle.

In the disclosure, the terms "solid phase support", "solid support", "solid phase carrier" and "solid carrier" can be used interchangeably and refer to a solid surface to which an antigen or antibody can be attached. The solid phase support used in the disclosure is not particularly limited, and commercial solid phase supports and any solid phase supports that is useful for immunodetection can all be used in the disclosure. Exemplary solid phase supports may be a magnetic bead (such as a superparamagnetic microsphere), an ELISA Plate, a plastic plate, a plastic tube, a latex bead, an agarose bead, a glass, a nitrocellulose membrane, a nylon membrane, a silica plate or a microchip, but the disclosure is not limited thereto.

Labels that can be used in the disclosure are well known to a person skilled in the art and can be, for example, enzymes, such as oxidase, microperoxidase, horseradish peroxidase, alkaline phosphatase (ALP), β-galactosidase, glucose oxidase and glucose 6-phosphate dehydrogenase; fluorescent substances, such as fluorescein isothiocyanate, tetramethylrhodamine isothiocyanate, fluorescein, rhodamine, europium and green fluorescent protein; chemiluminescent substances, such as luminol, isoluminol, phenanthridinium and acridinium ester; coenzymes, such as NAD; biotins; and radioactive substances, such as ³⁵S, ¹⁴C, ³²P, ¹³¹I and ¹²⁵I, but the disclosure is not limited thereto.

In the first reagent of the kit of the disclosure, at least one of the antigens is respectively coated on a solid phase support, and the antigens can be independently selected from one or more conservative proteins of a pathogen, or a fragment, native or recombinant, or synthetic, thereof. Preferably, the antigens are independently selected from one or more conservative fragments that predominate in a pathogen.

In some embodiments, the antigens in the first reagent may be present in the form of a polymer, an antigen fragment or a peptide.

In one exemplary embodiment, when the detection object is an anti-HCV antibody, the antigens in the first reagent of the disclosure may be selected from at least one of HCV core antigen, HCV NS3 antigen, HCV NS4 antigen and HCV NS5 antigen, or a fusion antigen of two or more of the above-mentioned antigens; preferably, the antigens are selected from at least one of HCV core antigen and HCV NS3 antigen; and more preferably, the antigens simultaneously contain HCV core antigen, HCV NS3 antigen, HCV NS4 antigen and HCV NS5 antigen. In a specific embodiment, in the first reagent, the HCV core antigen may be present at a concentration of about 1.5 µg/mL to about 4 µg/mL, the HCV NS3 antigen may be present at a concentration of about 0.5 µg/mL to about 2 µg/mL, the HCV NS4 antigen may be present at a concentration of about 0.05 µg/mL to about 0.2 µg /mL, and the HCV NS5 antigen may be present at a concentration of about 0.01 µg/mL to about 0.05 µg/mL.

In another exemplary embodiment, when the detection object is an anti-*Treponema pallidum* antibody, the antigens in the first reagent of the disclosure are selected from at least one of TP15 antigen, TP17 antigen, TP47 antigen and TP45 antigen, or a fusion antigen of two or more of the above-mentioned antigens; preferably, the antigens are selected from at least one of TP15 antigen, TP17 antigen and TP47 antigen; and more preferably, the antigens simultaneously contain TP15 antigen, TP17 antigen and TP47 antigen.

The antigen and antibody in the first reagent of the disclosure may be coated on a solid phase support by means of a conventional technique in the art (e.g., conjugation).

In the disclosure, the antigen and antibody coated on the solid phase support can be referred to as the antigen and antibody in the first reagent.

In the disclosure, the conjugate of the antigen or antibody with the solid phase support can be referred to as an antigen-solid phase carrier coating or an antibody-solid phase carrier coating. The solid phase carrier coating of the disclosure may be present in a conventional diluent containing proteins and surfactants and having buffering capacity.

In the second reagent of the kit of the disclosure, at least one of the antigens is labelled, and the labelled antigen can be independently selected from one or more conservative proteins of a pathogen, or a fragment thereof. Preferably, the antigens are independently selected from one or more conservative fragments that predominate in a pathogen.

In some embodiments, the antigens in the second reagent may be present in the form of a polymer, an antigen fragment or a peptide.

In one exemplary embodiment, when the detection object is an HCV antibody, the antigens in the second reagent of the disclosure may be selected from at least one of HCV core antigen, HCV NS3 antigen, HCV NS4 antigen and HCV NS5 antigen, or a fusion antigen of two or more of the above-mentioned antigens; preferably, the antigens are selected from at least one of HCV core antigen and HCV NS3 antigen; and more preferably, the antigens simultaneously contain HCV core antigen, HCV NS3 antigen, HCV NS4 antigen and HCV NS5 antigen. In a specific embodiment, in the second reagent, the HCV core antigen may be present at a concentration of about 0.3 µg/mL to about 1.5 µg/mL, the HCV NS3 antigen may be present at a concentration of about 0.1 µg/mL to about 1 µg/mL, the HCV NS4 antigen may be present at a concentration of about 0.01 µg/mL to about 0.1 µg/mL, and the HCV NS5 antigen may be present at a concentration of about 0.005 µg/mL to about 0.02 µg/mL.

In another exemplary embodiment, when the detection object is a *Treponema pallidum* antibody, the antigens in the second reagent of the disclosure are selected from at least one of TP15 antigen, TP17 antigen, TP47 antigen and TP45 antigen, or a fusion antigen of two or more of the above-mentioned antigens; preferably, the antigens are selected from at least one of TP15 antigen, TP17 antigen and TP47 antigen; and more preferably, the antigens simultaneously contain TP15 antigen, TP17 antigen and TP47 antigen.

The antigen and antibody in the second reagent of the disclosure can be linked to a label via a conventional technique in the art (e.g., chemical bonding).

In the disclosure, the labelled antigen and labelled antibody can be referred to as the antigen and antibody in the second reagent.

In the disclosure, the conjugate of the antigen or antibody with the label can be referred to as an antigen-label conjugate or an antibody-label conjugate. The label conjugate of the disclosure can be present in a conventional diluent containing proteins and surfactants and having buffering capacity.

The antibody in the kit of the disclosure may be a monoclonal antibody or a polyclonal antibody. In some embodiments, the antibodies in the first reagent and the second reagent may be an antibody fragment, which generally include at least a portion of the antigen binding region, the light chain and/or heavy chain variable region, and one or more (e.g., six) CDRs of an antibody. Examples of antibody fragments include, but are not limited to, Fab, Fab', F(ab')₂ and Fv fragments.

The kit of the disclosure may further include a third reagent containing a blocking agent, and the blocking agent may contain one or more components selected from the group consisting of: skimmed milk powder, BSA, gelatin, serum, casein, ovalbumin, animal IgGs and surfactants (such as Tween-20).

The occurrence of non-specific binding may be prevented by adding the third reagent containing the blocking agent, thereby improving the anti-interference capacity of the kit of the disclosure.

The kit of the disclosure may further include a fourth reagent containing a reducing agent, and the reducing agent may contain one or more components selected from the group consisting of: DTT and β-mercaptoethanol.

The antigen conformation may be maintained by adding the fourth reagent containing the reducing agent, thereby improving the sensitivity of the kit of the disclosure.

Unless otherwise specified, the wordings "first", "second", "third", "fourth" etc. in the disclosure are only used to distinguish a plurality of similar elements, and are not intended to indicate any difference in importance, order, etc. among elements.

Those skilled in the art can understand that, in addition to the first reagent and the second reagent, the kit of the disclosure may also include a sample diluent, a washing buffer, a quality control substance and/or a calibrator.

The wordings of detecting, measuring, assessing, assaying or testing etc. in the disclosure can be applied for quantification, semi-quantification, and qualification as intended.

In a second aspect, the disclosure provides a method for in vitro detecting an antibody against infectious diseases, including:
1) mixing and incubating a sample from a subject with a first reagent and a second reagent,
2) obtaining a signal value associated with a label,
3) determining a detection result according to the signal value;
   the first reagent comprises at least one antigen and an anti-human IgM antibody, the antigen and the anti-human IgM antibody are coated on a solid phase support, and
   the second reagent includes at least one labelled antigen and a labelled anti-human IgG antibody;
   and at least one antigen of the at least one antigen coated on a solid phase support and at least one antigen of the at least one labelled antigen can bind to the same IgG antibody or the same IgM antibody in the sample, that is to say, the at least one antigen coated on a solid phase support, the antibody to be detected and the at least one labelled antigen may form a double antigens sandwich complex.

In an alternative method of the disclosure, including:
1) mixing and incubating a sample from a subject with a first reagent and a second reagent,
2) obtaining a signal value associated with a label,
3) determining a detection result according to the signal value;
   the first reagent comprises at least one antigen and an anti-human IgG antibody, the antigen and the anti-human IgG antibody are coated on a solid phase support, and
   the second reagent includes at least one labelled antigen and a labelled anti-human IgM antibody;
   and at least one antigen of the at least one antigen coated on a solid phase support and at least one antigen of the at least one labelled antigen can bind to the same IgG antibody or the same IgM antibody in the sample, that is to say, the at least one antigen coated on a solid phase support, the antibody to be detected and at least one antigen of the at least one labelled antigen may form a double antigens sandwich complex.

The IgG antibody and the IgM antibody of pathogens of infectious diseases may be detected simultaneously by using the method of the disclosure. IgG is an indication of chronic, current or recurrent, or recovered infection, and IgM is an indication of acute infection. Therefore, the method of the disclosure can differentially detect both chronic infection and acute infection. In addition, the method of the disclosure overcomes the defects of traditional detection methods, and takes into account the sensitivity and specificity of a detection.

In step 1), the sample and the first reagent may be first added to a reaction vessel, incubated for a period of time, and washed; and the second reagent is then added to the reaction vessel, incubated for a period of time, and washed. Alternatively, the sample, the first reagent and the second reagent may be added to the reaction vessel simultaneously, incubated for a period of time, and washed. Alternatively, the sample and the second reagent may be added to the reaction vessel and incubated for a period of time (without washing); and the first reagent is then added to the reaction vessel, incubated for a period of time, and washed.

In step 2), the process of obtaining the signal value associated with the label is well known to those skilled in the art. For example, when alkaline phosphatase is used as a label, the luminescent substrate 3-(2-spiroadamantane)-4-methoxy-4-(3-phosphoryloxy)-phenyl-1,2-dioxetane (AMPPD) may be added to a reaction vessel and decomposed by the alkaline phosphatase to generate chemiluminescence, and finally the number of photons generated by the reaction is measured by a photomultiplier.

In step 2), a commercial detection instrument may also be used to measure the signal value corresponding to the label. For example, Mindray fully automated chemiluminescence instrument CL series may be used.

In a preferred embodiment, step 1) includes mixing and incubating the sample from the subject, the first reagent, the second reagent and the third reagent of the disclosure. For example, the sample, the first reagent and the third reagent may be first added to a reaction vessel, incubated for a period of time, and washed; and the second reagent and the third reagent is then added to the reaction vessel, incubated for a period of time, and washed. Alternatively, the sample, the first reagent, the second reagent and the third reagent may be added to the reaction vessel simultaneously, incubated for a period of time, and washed. Alternatively, the sample, the second reagent and the third reagent may be added to the reaction vessel and incubated for a period of time (without washing); and the first reagent and the third reagent are then added to the reaction vessel, incubated for a period of time, and washed.

In another preferred embodiment, step 1) includes mixing and incubating the sample from the subject, the first reagent, the second reagent and the fourth reagent of the disclosure. For example, the sample, the first reagent and the fourth reagent may be first added to a reaction vessel, incubated for a period of time, and washed; and the second reagent is then added to the reaction vessel, incubated for a period of time, and washed. Alternatively, the sample, the first reagent, the second reagent and the fourth reagent may be added to the reaction vessel simultaneously, incubated for a period of time, and washed. Alternatively, the sample, the second reagent and the fourth reagent may be added to the reaction vessel and incubated for a period of time (without washing); and the first reagent is then added to the reaction vessel, incubated for a period of time, and washed.

In yet another preferred embodiment, step 1) includes mixing and incubating the sample from the subject, the first reagent, the second reagent, the third reagent of the disclosure and the fourth reagent of the disclosure. For example, the sample, the first reagent, the third reagent and the fourth reagent may be first added to a reaction vessel, incubated for a period of time, and washed; and the second reagent and the third reagent is then added to the reaction vessel, incubated for a period of time, and washed. Alternatively, the sample, the first reagent, the second reagent, the third reagent and the fourth reagent may be added to the reaction vessel simultaneously, incubated for a period of time, and washed. Alternatively, the sample, the second reagent, the third reagent and the fourth reagent may be added to the reaction vessel and incubated for a period of time (without washing); and the first reagent and the third reagent are then added to the reaction vessel, incubated for a period of time, and washed.

In addition, the in vitro detection method provided in the second aspect of the disclosure may be realized by using the kit provided in the first aspect of the disclosure.

In the disclosure, the sample is a blood sample from a subject, e.g., blood, serum or plasma.

### BRIEF DESCRIPTION OF THE DRAWINGS

FIG. 1 is a schematic diagram of the reaction principle of the kit of the disclosure.

### DESCRIPTION OF THE PREFERRED EMBODIMENTS

The technical solutions of the embodiments of the disclosure will be described below clearly and completely in conjunction with the embodiments and accompanying drawings of the disclosure. Obviously, the embodiments described are merely some of the embodiments of the disclosure rather than all the embodiments. Based on the embodiments in the disclosure, all the other embodiments that would have been obtained by those of ordinary skill in the art without any inventive effort shall fall within the scope of protection of the disclosure.

### Reagent preparation

First reagent Ra:
   A volume of V1/Dₓ (x = 1, 2 ...) (mL) of "magnetic beads coated with antigen" and "magnetic beads coated with anti-human IgM antibody" were respectively measured off using a pipette or a cylinder and added to a magnetic bead-coated tube to replace a supernatant; that is, after the magnetic separation was completed, the supernatant was suctioned off, and then an equal volume of a magnetic bead coating diluent was added and mixed evenly; the magnetic beads were mixed evenly and then added to a liquid preparation bottle and diluted to V1 (mL)volume by the magnetic bead coating diluent; and the mixture was stirred until the magnetic bead suspension was completely mixed to obtain the first reagent Ra; Dₓ is the dilution degree of "magnetic bead coated e with antigen" and "magnetic bead coated with anti-human IgM antibody"; and the magnetic bead coating diluent is a conventional diluent with buffering capacity and contains a protein and a surfactant.
Second reagent Rb:
   A certain volume of a diluent for an enzyme-labelled conjugate was measured off using a suitable cylinder and added to a liquid preparation bottle, and V2/D_{y} (y = 1, 2...) (mL) volume of "antigen-enzyme label conjugate" and "anti-human IgG antibody-enzyme label conjugate" were measured off using a pipette or a cylinder and added to the liquid preparation bottle and diluted to V2 (mL) volume; the solution was stirred with a stirrer to fully dissolve and mix evenly; the resulting solution was sealed, then placed at 22°C and left to equilibrate for 23 hours to 25 hours; after the equilibration was completed, the prepared solution was filtered using a suitable filter with a pore size of 0.22 µm, and the filtrate was collected to obtain the second reagent Rb; D_{y} is the dilution degree of each "antigen-enzyme label conjugate" and "anti-human IgG antibody-enzyme label conjugate"; and the diluent of the enzyme label conjugate is a conventional diluent with buffering capacity and contains a protein and a surfactant.
Third reagent Rc:
   a diluent has buffering capacity and contains a blocking agent and a surfactant.
Fourth reagent Rd:
   a diluent has buffering capacity and contains a reducing agent.

### Pathogen detection method

Step 1: the sample, the third reagent, the fourth reagent and the first reagent were added to a reaction tube and incubated at 37°C for 10 minutes, so that the antigen and anti-human IgM antibody, which were coated on the solid phase of magnetic beads, were fully bound to IgG and IgM antibodies in the sample; and after incubation was complete, the solid phase of magnetic beads was placed in a magnetic field, attracted and adsorbed, the substances bound to the solid phase of magnetic beads were retained, and other unbound substances were washed off.

Step 2: the third reagent and the second reagent were added to the reaction tube; after the mixture was incubated at 37°C for 10 minutes, the antigen and anti-human IgG antibody on the enzyme label conjugate were bound to the IgG and IgM antibodies captured on the magnetic beads to form a sandwich complex; and after incubation in the reaction tube was completed, the complex was attracted and adsorbed by the magnetic field, and other unbound substances were washed off.

Step 3: a chemiluminescent substrate was added to the reaction tube to generate chemiluminescence; and the number of photons produced by the reaction was then measured by a photomultiplier to obtain a chemiluminescence value of the sample.

Example 1 Preparation of HCV antibody detection kit

The kits used in the example were prepared as described in Table 1 below and "Reagent preparation".

**Table 1**

| Kit | First reagent (VI = 8 mL) | Second reagent (V2 = 8 mL) | Third reagent (15 mL) | Fourth reagent (8 mL) |
|---|---|---|---|---|
| Kit 1-1 | Magnetic bead coated with HCV core antigen (D = 100); | HCV core antigen labelled with ALP (D = 500); | 1% BSA 0.05% Tween -20 3% skimmed milk powder | 10 mM DTT |
| | Magnetic bead coated with HCV NS3 antigen (D = 100); | HCV NS3 antigen labelled with ALP (D = 500); | | |
| | Magnetic bead coated with HCV NS4 antigen (D = 200); | HCV NS4 antigen labelled with ALP (D = 800); | | |
| | Magnetic bead coated with HCV NS5 antigen (D = 400); | HCV NS5 antigen labelled with ALP (D = 1000); | | |
| | Magnetic bead coated with anti-human IgM antibody (D = 100). | Anti-human IgG antibody labelled with ALP (D = 300). | | |
| Kit 1-2 | Magnetic bead coated with HCV core antigen (D = 100); | Anti-human IgG antibody labelled with ALP (D = 300). | Ditto | Ditto |
| | Magnetic bead coated with HCV NS3 antigen (D = 100); | | | |
| | Magnetic bead coated with HCV NS4 antigen (D = 200); | | | |
| | Magnetic bead coated with HCV NS5 antigen (D = 400). | | | |
| Kit 1-3 | Magnetic bead coated with HCV core antigen (D = 100); | HCV core antigen labelled with ALP (D = 500); | Ditto | Ditto |
| | Magnetic bead coated with HCV NS3 antigen (D = 100); | HCV NS3 antigen labelled with ALP (D = 500); | | |
| | Magnetic bead coated with HCV NS4 antigen (D = 200); | HCV NS4 antigen labelled with ALP (D = 800); | | |
| | Magnetic bead coated with HCV NS5 antigen (D = 400). | HCV NS5 antigen labelled with ALP (D = 1000). | | |
| Kit 1-4 | Magnetic bead coated with anti-human IgM antibody (D = 100). | HCV core antigen labelled with ALP (D = 500); | Ditto | Ditto |
| | | HCV NS3 antigen labelled with ALP (D = 500); | | |
| | | HCV NS4 antigen labelled with ALP (D = 800); | | |
| | | HCV NS5 antigen labelled with ALP (D = 1000). | | |
| Kit 1-5 | Magnetic bead coated with HCV core antigen (D = 100); | HCV core antigen labelled with ALP (D = 500); | Ditto | Ditto |
| | Magnetic bead coated with HCV NS3 antigen (D = 100); | HCV NS3 antigen labelled with ALP (D = 500); | | |
| | Magnetic bead coated with HCV NS4 antigen (D = 200); | HCV NS4 antigen labelled with ALP (D = 800); | | |
| | Magnetic bead coated with HCV NS5 antigen (D = 400). | HCV NS5 antigen labelled with ALP (D = 1000); | | |
| | | Anti-human IgG antibody labelled with ALP (D = 300). | | |
| Kit 1-6 | Magnetic bead coated with HCV core antigen (D = 100); | HCV core antigen labelled with ALP (D = 500); | Ditto | Ditto |
| | Magnetic bead coated with HCV NS3 antigen (D = 100); | HCV NS3 antigen labelled with ALP (D = 500); | | |
| | Magnetic bead coated with HCV NS4 antigen (D = 200); | HCV NS4 antigen labelled with ALP (D = 800); | | |
| | Magnetic bead coated with HCV NS5 antigen (D = 400); | HCV NS5 antigen labelled with ALP (D = 1000). | | |
| | Magnetic bead coated with anti-human IgM antibody (D = 100). | | | |
| Kit 1-7 | Magnetic bead coated with HCV core antigen (D = 100); | HCV core antigen labelled with ALP (D = 500); | Ditto | Ditto |
| | Magnetic bead coated with HCV NS3 antigen (D = 100); | HCV NS3 antigen labelled with ALP (D = 500); | | |
| | Magnetic bead coated with HCV NS4 antigen (D = 200); | Anti-human IgG antibody labelled with ALP (D = 300). | | |
| | Magnetic bead coated with HCV NS5 antigen (D = 400); | | | |
| | Magnetic bead coated with anti-human IgM antibody (D = 100). | | | |

### Example 2 Detection of serum panel of HCV antibody

According to "Pathogen detection method", three seroconversion panels (9041, 10165 and 10185, purchased from Zeptometrix) were tested, and the COI results for each sample are as shown in Table 2 below.

**Table 2**

| | Kit 1-1 | Kit1-2 | Kit 1-3 | Kit 1-4 | Kit 1-5 | Kit 1-6 | Kit 1-7 |
|---|---|---|---|---|---|---|---|
| Sample | COI | COI | COI | COI | COI | COI | COI |
| 9041-1 | 0.17 | 0.33 | 0.11 | 0.16 | 0.13 | 0.08 | 0.13 |
| 9041-2 | 0.18 | 0.32 | 0.11 | 0.18 | 0.16 | 0.10 | 0.15 |
| 9041-3 | 0.15 | 0.36 | 0.10 | 0.15 | 0.14 | 0.09 | 0.16 |
| 9041-4 | 0.18 | 0.46 | 0.12 | 0.25 | 0.17 | 0.08 | 0.16 |
| 9041-5 | **27.04** | **49.47** | **3.26** | 0.49 | **19.15** | **4.18** | **14.33** |
| 9041-6 | **29.80** | **53.03** | **4.10** | 0.62 | **20.02** | **5.13** | **19.56** |
| 9041-7 | **35.73** | **56.95** | **4.27** | 0.82 | **22.51** | **5.20** | **23.10** |
| 9041-8 | **35.22** | **59.01** | **4.55** | **1.10** | **22.02** | **6.18** | **27.84** |
| 10165-1 | 0.33 | 0.15 | 0.14 | 0.16 | 0.12 | 0.09 | 0.24 |
| 10165-2 | 0.26 | 0.13 | 0.20 | 0.14 | 0.11 | 0.08 | 0.25 |
| 10165-3 | 0.26 | 0.13 | 0.14 | 0.15 | 0.10 | 0.09 | 0.25 |
| 10165-4 | 0.28 | 0.14 | 0.14 | 0.15 | 0.11 | 0.09 | 0.26 |
| 10165-5 | **1.04** | 0.47 | 0.15 | 0.42 | 0.19 | 0.10 | **1.01** |
| 10165-6 | **19.92** | **11.79** | 0.55 | **1.56** | **5.27** | **2.57** | **16.77** |
| 10165-7 | **22.51** | **14.23** | **1.79** | **3.07** | **6.36** | **4.71** | **19.03** |
| 10165-8 | **34.09** | **24.82** | **2.77** | **4.77** | **10.73** | **4.50** | **28.15** |
| 10165-9 | **38.55** | **29.13** | **4.80** | **4.64** | **12.33** | **5.30** | **30.24** |
| 10185-1 | **1.08** | 0.15 | **1.02** | 0.61 | 0.87 | 0.88 | **1.03** |
| 10185-2 | **24.95** | **10.73** | **1.22** | 0.76 | **10.77** | **1.00** | **20.48** |
| 10185-3 | **31.38** | **12.54** | **1.25** | 0.90 | **13.59** | **1.05** | **27.67** |
| 10185-4 | **36.47** | **14.34** | **1.07** | 0.71 | **16.48** | 0.87 | **31.52** |
| 10185-5 | **31.99** | **13.66** | 0.95 | 0.62 | **14.54** | 0.79 | **30.87** |

In the disclosure, COI (Cutoff index) refers to the relative luminescence unit (RLU) value of a test sample, that is, the ratio of a chemiluminescence signal value to a threshold value (Cutoff), where COI ≥ 1 represents that the test sample is a reactive sample, and COI < 1 represents that the test sample is a non-reactive sample. For qualitative detection methods, the threshold value (cutoff value) is a cut-off value for determining whether the test result is reactive or non-reactive.

It can be seen from Table 2 that kit 1-1 (i.e., the kit of the disclosure) has a comparable detection rate to kit 1-2 (indirect method) and kit 1-5 (double antigens sandwich + indirect method), a higher COI value and a more obvious gradient of detected samples; and kit 1-3 (double antigens sandwich), kit 1-4 (immunocapture method) and kit 1-6 (double antigens sandwich + immunocapture method) have fewer detected samples, most of which have lower COI (< 5), and the gradient of the detected samples is not obvious. It can be seen that, in terms of serum panel sensitivity, the kit/method of the disclosure is superior to the kit/method based on the principle of the double antigens sandwich, the kit/method based on the principle of the immunocapture method, and the kit/method based on a combination of the two (double antigens sandwich + immunocapture method). In addition, kit 1-7 has the similar performance in terms of serum panel sensitivity to kit 1-1.

### Example 3 Detection of HCV-IgM antibody positive sample

According to "Pathogen detection method", 31 HCV-IgM antibody positive samples (which are the samples treated with IgG/RF adsorption reagent (purchased from Oumeng, Germany) and then confirmed by Roche Anti-HCV kit) were tested, and the results are as shown in Table 3 below.

**Table 3**

| | Kit 1-1 | Kit 1-2 | Kit 1-3 | Kit 1-4 | Kit 1-5 | Kit 1-6 | Kit 1-7 |
|---|---|---|---|---|---|---|---|
| Sample | COI | COI | COI | COI | COI | COI | COI |
| 1 | **2.77** | 0.59 | **1.14** | **5.33** | **1.12** | **2.39** | **2.25** |
| 2 | **1.94** | 0.82 | **1.10** | **2.55** | **1.15** | **1.05** | **1.58** |
| 3 | **2.41** | 0.83 | **1.11** | **4.36** | **1.10** | **1.80** | **1.16** |
| 4 | **2.18** | 0.62 | **1.02** | **3.62** | **1.21** | **1.62** | **1.77** |
| 5 | **6.76** | 0.99 | 1.00 | **8.99** | **1.13** | **7.00** | **5.44** |
| 6 | **1.95** | 0.53 | **1.26** | **3.24** | **1.25** | **1.41** | **1.59** |
| 7 | **2.73** | 0.77 | **1.16** | **5.35** | **1.12** | **2.36** | **2.21** |
| 8 | **2.21** | 0.57 | **1.23** | **3.76** | **1.20** | **1.66** | **1.80** |
| 9 | **2.36** | 0.81 | **1.15** | **4.12** | **1.11** | **1.73** | **1.92** |
| 10 | **1.93** | 0.93 | **1.11** | **2.74** | **1.08** | **1.11** | **1.57** |
| 11 | **6.80** | **1.47** | **1.15** | **8.31** | **1.21** | **6.84** | **5.47** |
| 12 | **2.61** | **1.43** | 0.95 | **5.16** | **1.08** | **2.24** | **2.12** |
| 13 | **5.59** | **1.45** | 0.85 | **7.73** | 0.96 | **5.48** | **4.50** |
| 14 | **1.86** | 0.68 | 0.94 | **2.81** | 0.89 | **1.20** | **1.52** |
| 15 | **2.32** | 0.81 | 0.88 | **4.42** | 0.88 | **1.91** | **1.19** |
| 16 | **2.36** | **1.06** | **1.02** | **4.53** | **1.04** | **2.04** | **1.92** |
| 17 | **1.93** | **1.39** | 0.93 | **3.11** | **1.05** | **1.04** | **1.57** |
| 18 | **2.55** | 0.81 | 0.99 | **4.86** | 0.89 | **2.11** | **2.07** |
| 19 | **3.91** | 0.88 | 0.93 | **3.32** | 0.86 | **1.23** | **3.16** |
| 20 | **1.67** | 0.88 | **1.08** | **2.51** | **1.06** | **1.01** | **1.37** |
| 21 | **1.58** | 0.70 | 0.87 | **2.17** | 0.82 | 0.83 | **1.29** |
| 22 | **2.14** | **1.24** | 0.87 | **3.74** | 0.89 | **1.49** | **1.74** |
| 23 | **1.50** | 0.62 | 0.97 | **2.32** | 0.89 | 0.89 | **1.23** |
| 24 | **2.69** | 0.70 | 0.88 | **2.78** | 0.87 | **1.09** | **2.18** |
| 25 | **1.66** | 0.89 | **1.03** | **2.63** | 0.98 | **1.03** | **1.36** |
| 26 | **2.62** | 0.75 | 0.90 | **2.39** | 0.84 | 0.82 | **2.13** |
| 27 | **1.47** | 0.79 | 0.99 | **2.28** | 0.93 | 0.83 | **1.21** |
| 28 | **4.64** | 0.82 | 0.92 | **1.73** | **1.96** | 0.63 | **3.74** |
| 29 | **3.56** | 0.72 | **1.14** | **2.13** | **1.05** | 0.92 | **2.88** |
| 30 | **1.58** | **1.22** | 0.84 | **2.61** | 0.84 | 0.96 | **1.09** |
| 31 | **2.84** | **1.03** | **1.03** | **5.50** | **1.01** | **2.38** | **2.30** |

It can be seen from Table 3 that, in the case of HCV-IgM positive sample detection, all the samples are detected by kit 1-1 (the kit of the disclosure) and kit 1-4 (immunocapture method); less than half of the samples are detected positive by kit 1-2 (indirect method) and kits 1-3 (double antigens sandwich), and the COI values are all relatively low, most of which are around 1; kit 1-5 (double antigens sandwich + indirect method) and kit 1-6 (double antigens sandwich + immunocapture method) have an increase in the detected values compared with kit 1-2 and kit 1-3, but there are still samples that failed to be detected. It can be seen that, in terms of the sensitivity of detecting HCV IgM positive samples, the kit/method of the disclosure is superior to the kit/method based on the principle of the indirect method, the kit/method based on the principle of the double antigens sandwich, and the kit/method based on a combination of the two (double antigens sandwich + indirect method); moreover, a combination of the immunocapture method and the double antigens sandwich method may reduce the detection rate of HCV IgM positive samples (the detection rate of combination 1-6 is weaker than that of combination 1-4). In addition, kit 1-7 has basically the same performance in terms of the detection rate of HCV-IgM antibody positive samples as kit 1-1.

### Example 4 Detection of HCV antibody negative sample

According to "Pathogen detection method", 500 HCV antibody negative samples (from hospital diagnosis results) were tested, and the statistical results are as shown in Table 4 below.

**Table 4**

| | Kit 1-1 | Kit 1-2 | Kit 1-3 | Kit 1-4 | Kit 1-5 | Kit 1-6 |
|---|---|---|---|---|---|---|
| Total number of samples | 500 | 500 | 500 | 500 | 500 | 500 |
| Number of false positive samples | 1 | 3 | 1 | 5 | 2 | 3 |
| False positive probability | 0.2% | 0.6% | 0.2% | 1% | 0.4% | 0.6% |

It can be seen from Table 4 that in the case of HCV antibody negative sample detection, kit 1-1 (the kit the disclosure) and kit 1-3 (double antigens sandwich) have comparable false positive occurrence, which is 0.2%; combination 1-2 (indirect method) and kit 1-4 (immunocapture method) have higher false positive occurrence, which are 0.6% and 1%, respectively; kits combining two detection formats: kit 1-5 (double antigens sandwich + indirect method) and combination 1-6 (double antigens sandwich + immunocapture method) have reduced false positive occurrence compared with kit 1-2 (indirect method) and kit 1-4 (immunocapture method), but higher false positive occurrence than kit 1-1 (the kit of the disclosure) and kit 1-3 (double antigens sandwich). It can be seen that in terms of the specificity of detecting HCV antibody negative samples, the kit/method of the disclosure is superior to the kit/method based on the principle of the indirect method or the principle of the immunocapture method and the kit/method based on a combination of either the indirect method or the immunocapture method and the double antigens sandwich method.

### Example 5 Preparation of Treponema pallidum antibody detection kit

The kits used in the example were prepared as described in Table 5 below and "Reagent preparation".

**Table 5**

| Kit | First reagent (VI = 8 mL) | Second reagent (V2 = 8 mL) | Third reagent (15 mL) | Fourth reagent (8 mL) |
|---|---|---|---|---|
| Kit 2-1 | Magnetic bead coated with TP15 antigen (D = 100); Magnetic bead coated with TP17 antigen (D = 100); Magnetic bead coated with TP47 antigen (D = 200); Magnetic bead coated with anti-human IgM antibody (D = 100). | TP15 antigen labelled with ALP (D = 500); TP17 antigen labelled with ALP (D = 500); TP47 antigen labelled with ALP (D = 800); Anti-human IgG antibody labelled with ALP (D = 300). | 1% BSA 0.05% Tween -20 3% casein 0.001% SDS | 10 mM DTT |
| Kit 2-2 | Magnetic bead coated with TP15 antigen (D = 100); Magnetic bead coated with TP17 antigen (D = 100); Magnetic bead coated with TP47 antigen (D = 200). | Anti-human IgG antibody labelled with ALP (D = 300). | Ditto | Ditto |
| Kit 2-3 | Magnetic bead coated with TP15 antigen (D = 100); Magnetic bead coated with TP17 antigen (D = 100); Magnetic bead coated with TP47 antigen (D = 200). | TP15 antigen labelled with ALP (D = 500); TP17 antigen labelled with ALP (D = 500); TP47 antigen labelled with ALP (D = 800). | Ditto | Ditto |
| Kit 2-4 | Magnetic bead coated with anti-human IgM antibody (D = 100). | TP15 antigen labelled with ALP (D = 500); TP17 antigen labelled with ALP (D = 500); TP47 antigen labelled with ALP (D = 800). | Ditto | Ditto |
| Kit 2-5 | Magnetic bead coated with TP15 antigen (D = 100); Magnetic bead coated with TP17 antigen (D = 100); Magnetic bead coated with TP47 antigen (D = 200). | TP15 antigen labelled with ALP (D = 500); TP17 antigen labelled with ALP (D = 500); TP47 antigen labelled with ALP (D = 800); Anti-human IgG antibody labelled with ALP (D = 300). | Ditto | Ditto |
| Kit 2-6 | Magnetic bead coated with TP15 antigen (D = 100); | TP15 antigen labelled with ALP (D = 500); | Ditto | Ditto |
| | Magnetic bead coated with TP17 antigen (D = 100); Magnetic bead coated with TP47 antigen (D = 200); Magnetic bead coated with anti-human IgM antibody (D = 100). | TP17 antigen labelled with ALP (D = 500); TP47 antigen labelled with ALP (D = 800). | | |

### Example 6 Detection of serum panel of Treponema pallidum antibody

According to "Pathogen detection method", two serum panels (0615-0017 and 0820-0300, purchased from SeraCare) were tested, and the results are shown in Table 6 below.

**Table 6**

| | Kit 2-1 | Kit 2-2 | Kit 2-3 | Kit 2-4 | Kit 2-5 | Kit 2-6 |
|---|---|---|---|---|---|---|
| Sample | COI | COI | COI | COI | COI | COI |
| 0615-0017-01 | 0.17 | 0.14 | 0.05 | 0.42 | 0.18 | 0.35 |
| 0615-0017-02 | 0.47 | 0.12 | 0.04 | 0.44 | 0.19 | 0.39 |
| 0615-0017-03 | 0.42 | 0.12 | 0.03 | 0.41 | 0.17 | 0.41 |
| 0615-0017-04 | 0.58 | 0.13 | 0.03 | 0.40 | 0.21 | 0.42 |
| 0615-0017-05 | **1.98** | 0.16 | 0.13 | 0.68 | 0.23 | 0.73 |
| 0615-0017-06 | **3.07** | 0.51 | **1.04** | **1.17** | 0.78 | **1.23** |
| 0615-0017-07 | **3.68** | 0.80 | **2.11** | **1.26** | **1.21** | **1.31** |
| 0615-0017-08 | **4.20** | 0.97 | **5.10** | **1.51** | **1.68** | **2.49** |
| 0615-0017-09 | **11.20** | **1.32** | **12.40** | **1.87** | **2.05** | **3.88** |
| 0820-0300-01 | **10.14** | **1.91** | **5.37** | 0.73 | **2.19** | **1.08** |
| 0820-0300-02 | **2.92** | **3.16** | **1.10** | 0.85 | **2.15** | 0.98 |
| 0820-0300-03 | **4.10** | **4.48** | **2.22** | 0.77 | **1.86** | **1.03** |
| 0820-0300-04 | **3.31** | 0.89 | **2.15** | 0.71 | **1.36** | 0.95 |
| 0820-0300-05 | **3.54** | **1.05** | **1.33** | 0.73 | 0.98 | **1.09** |
| 0820-0300-06 | **2.90** | **1.38** | **1.21** | 0.79 | 0.97 | 1.00 |
| 0820-0300-07 | **10.20** | **3.11** | **7.29** | 0.79 | **1.04** | **1.05** |
| 0820-0300-08 | **2.62** | **1.05** | **1.21** | 0.74 | **1.04** | **1.17** |
| 0820-0300-09 | **8.11** | **1.15** | **6.41** | **3.19** | **1.10** | **1.02** |
| 0820-0300-10 | **2.34** | **1.14** | **1.13** | 0.96 | **1.05** | **1.02** |
| 0820-0300-11 | **3.28** | 0.91 | **1.13** | 0.78 | **1.02** | 0.96 |
| 0820-0300-12 | 0.07 | 0.26 | 0.06 | 0.48 | 0.15 | 0.21 |
| 0820-0300-13 | **4.15** | **1.07** | **2.26** | **1.72** | **1.02** | **2.10** |
| 0820-0300-14 | **2.90** | **1.07** | **1.14** | **1.69** | 0.95 | **2.06** |
| 0820-0300-15 | **3.61** | 0.80 | **1.34** | **1.46** | **1.02** | **1.79** |
| 0820-0300-16 | **3.28** | **1.29** | **3.17** | **1.06** | **1.02** | **1.33** |
| 0820-0300-17 | **2.65** | 0.68 | **1.28** | 0.68 | 0.96 | 0.89 |
| 0820-0300-18 | **2.83** | 1.00 | **1.20** | 0.75 | **2.03** | **3.78** |
| 0820-0300-19 | **2.32** | 0.74 | **1.48** | 0.73 | **1.24** | **1.22** |
| 0820-0300-20 | **8.16** | **1.05** | **5.09** | 0.73 | **1.00** | **1.01** |

It can be obtained from the results in Table 6 that the detection rates of serum panels of kit 2-1 (i.e., the kit of the disclosure) and kit 2-3 (double antigens sandwich method) are comparable; however, kit 2-1 shows a higher COI value and a more obvious gradient of detected samples than kit 2-3; the detection rates of kit 2-2 (indirect method), kit 2-4 (immunocapture method), kit 2-5 (double antigens sandwich + indirect method) and kit 2-6 (double antigens sandwich + immunocapture method) are weaker than those of kits 2-1 and 2-3; and most of the detected samples of kit 2-2 (indirect method), kit 2-4 (immunocapture method), kit 2-5 (double antigens sandwich + indirect method) and kit 2-6 (double antigens sandwich + immunocapture method) have lower COI (around 1), and the gradient of the detected samples is not obvious. It can be seen that, in terms of serum panel sensitivity, the kit/method of the disclosure is superior to the method based on the principle of the indirect method, the kit/method based on the principle of the immunocapture method and the kit/method based on a combination of either the indirect method or the immunocapture method and the double antigens sandwich (double antigens sandwich + indirect method and double antigens sandwich + immunocapture method).

### Example 7 Detection of Treponema pallidum antibody-IgM positive sample

According to "Pathogen detection method", 30 Treponema pallidum antibody-IgM positive samples (which are the samples treated with IgG/RF adsorption reagent (purchased from Oumeng, Germany) and then confirmed by Roche *Treponema pallidum* antibody kit) were tested, and the results are as shown in Table 7 below.

**Table 7**

| | Kit 2-1 | Kit 2-2 | Kit 2-3 | Kit 2-4 | Kit 2-5 | Kit 2-6 |
|---|---|---|---|---|---|---|
| Sample | COI | COI | COI | COI | COI | COI |
| 1 | **2.39** | 0.83 | 0.91 | **4.55** | 0.91 | **1.97** |
| 2 | **2.43** | **1.09** | **1.05** | **4.67** | **1.07** | **2.10** |
| 3 | **1.99** | **1.43** | 0.96 | **3.20** | **1.08** | **1.07** |
| 4 | **2.63** | 0.83 | **1.02** | **5.01** | 0.92 | **2.17** |
| 5 | **6.96** | **1.02** | **1.03** | **9.26** | **1.16** | **7.21** |
| 6 | **2.01** | 0.55 | **1.30** | **3.34** | **1.29** | **1.45** |
| 7 | **2.81** | 0.79 | **1.19** | **5.51** | **1.15** | **2.43** |
| 8 | **2.28** | 0.59 | **1.27** | **3.87** | **1.24** | **1.71** |
| 9 | **2.43** | 0.83 | **1.18** | **4.24** | **1.14** | **1.78** |
| 10 | **1.99** | 0.96 | **1.14** | **2.82** | **1.11** | 1.14 |
| 11 | **7.00** | **1.51** | **1.18** | **8.56** | **1.25** | **7.05** |
| 12 | **2.69** | **1.47** | 0.98 | **5.31** | **1.11** | **2.31** |
| 13 | **5.76** | **1.49** | 0.88 | **7.96** | 0.99 | **5.64** |
| 14 | **1.92** | 0.70 | 0.97 | **2.89** | 0.92 | **1.24** |
| 15 | **2.77** | 0.72 | 0.91 | **2.86** | 0.90 | **1.12** |
| 16 | **1.71** | 0.92 | **1.06** | **2.71** | **1.01** | **1.06** |
| 17 | **2.70** | 0.77 | 0.93 | **2.46** | 0.87 | 0.84 |
| 18 | **1.51** | 0.81 | **1.02** | **2.35** | 0.96 | 0.85 |
| 19 | **4.03** | 0.91 | 0.96 | **3.42** | 0.89 | **1.27** |
| 20 | **1.72** | 0.91 | **1.11** | **2.59** | **1.09** | **1.04** |
| 21 | **1.63** | 0.72 | 0.90 | **2.24** | 0.84 | 0.85 |
| 22 | **2.20** | **1.28** | 0.90 | **3.85** | 0.92 | **1.53** |
| 23 | **1.55** | 0.64 | 1.00 | **2.39** | 0.92 | 0.92 |
| 24 | **4.78** | 0.84 | 0.95 | **1.78** | **2.02** | 0.65 |
| 25 | **3.67** | 0.74 | **1.17** | **2.19** | **1.08** | 0.95 |
| 26 | **1.63** | **1.26** | 0.87 | **2.69** | 0.87 | 0.99 |
| 27 | **2.93** | **1.06** | **1.06** | **5.67** | **1.04** | **2.45** |
| 28 | **2.85** | 0.61 | **1.17** | **5.49** | **1.15** | **2.46** |
| 29 | **2.00** | 0.84 | **1.13** | **2.63** | **1.18** | **1.08** |
| 30 | **2.48** | 0.85 | **1.14** | **4.49** | **1.13** | **1.85** |

It can be seen from Table 7 that, in the case of *Treponema pallidum*-IgM positive sample detection, all the samples are detected by kit 2-1 (the kit of the disclosure) and kit 2-4 (immunocapture method); only about half of the samples are detected positive by kit 2-2 (indirect method) and kits 2-3 (double antigens sandwich), and the COI values are all relatively low, most of which are around 1; kit 2-5 (double antigens sandwich + indirect method) and kit 2-6 (double antigens sandwich + immunocapture method) have an increase in the detected number compared with kit 2-2 and kit 2-3, but there are still samples that failed to be detected. It can be seen that, in terms of the sensitivity for detecting *Treponema pallidum* antibody-IgM positive samples, the kit/method of the disclosure is superior to the method based on the principle of the indirect method, the kit/method based on the principle of the double antigens sandwich and the kit/method based on a combination of the two (double antigens sandwich + indirect method); moreover, a combination of the immunocapture method and the double antigens sandwich method may reduce the detection rate of *Treponema pallidum* antibody-IgM positive samples (the detection rate of kit 2-6 is weaker than that of kit 2-4). This conclusion is consistent with that in Example 3.

### Example 8 Detection of Treponema pallidum antibody negative samples

According to "Pathogen detection method", 500 *Treponema pallidum* antibody negative samples (from hospital diagnosis results) were tested, and the statistical results are as shown in Table 8 below.

**Table 8**

| | Kit 2-1 | Kit 2-2 | Kit 2-3 | Kit 2-4 | Kit 2-5 | Kit 2-6 |
|---|---|---|---|---|---|---|
| Total number of samples | 500 | 500 | 500 | 500 | 500 | 500 |
| Number of false positive samples | 0 | 1 | 0 | 2 | 1 | 1 |
| False positive probability | 0% | 0.2% | 0% | 0.4% | 0.2% | 0.2% |

It can be seen from Table 8 that in the case of *Treponema pallidum* antibody negative sample detection, neither kit 2-1 (the kit the disclosure) nor kit 2-3 (double antigens sandwich) have false positive samples; kit 2-2 (indirect method), kit 2-4 (immunocapture method) and kits combining the two detection formats, i.e., kit 2-5 (double antigens sandwich + indirect method) and kit 2-6 (double antigens sandwich + immunocapture method) all have a false positive occurrence of 0.2%-0.4%. It can be seen that in terms of the specificity for detecting *Treponema pallidum* antibody negative samples, the kit/method of the disclosure is superior to the kit/method based on the indirect method or the immunocapture method and the kit/method based on a combination of either the indirect method or the immunocapture method and the double antigens sandwich method.

## Claims

1. A kit for detecting antibodies against a pathogen or pathogens for infectious diseases in a human blood sample, comprising:
a first reagent containing at least one antigen and an anti-human IgM antibody, the antigen and the anti-human IgM antibody are coated on a solid phase support, and
a second reagent containing at least one labelled antigen and a labelled anti-human IgG antibody,
wherein the at least one antigen coated on the solid phase support and the at least one labelled antigen are capable of binding to a same IgG antibody or a same IgM antibody in the sample.

2. A kit for detecting antibodies against a pathogen or pathogens for infectious diseases in human blood sample, comprising:
a first reagent containing at least one antigen and an anti-human IgG antibody, the antigen and the anti-human IgG antibody are coated on a solid phase support, and
a second reagent containing at least one labelled antigen and a labelled anti-human IgM antibody,
wherein the at least one antigen coated on the solid phase support and the at least one labelled antigen are capable of binding to a same IgG antibody or a same IgM antibody in the sample.

3. The kit of claim 1 or 2, further comprising a third reagent, wherein the third reagent contains a blocking agent, preferably, the blocking agent contains one or more components selected from a group consisting of: skimmed milk powder, BSA, gelatin, serum, casein, ovalbumin, animal IgG and surfactant.

4. The kit of any one of claims 1 to 3, further comprising a fourth reagent, wherein the fourth reagent contains a reducing agent, preferably, the reducing agent contains one or more components selected from a group consisting of: DTT and β-mercaptoethanol.

5. The kit of any one of claims 1 to 4, wherein the kit is used for detection of an antibody produced in a subject infected by pathogen, wherein the pathogen is a virus or a spirochete, preferably, the virus is HCV, preferably, the spirochete is *Treponema pallidum.*

6. The kit of claim 5, wherein the kit is used for detection of an antibody produced in a subject infected by HCV; the at least one antigen coated on the solid phase support is selected from a group consisting of HCV core antigen, HCV NS3 antigen, HCV NS4 antigen and HCV NS5 antigen, which are coated on the solid phase support respectively; the at least one labelled antigen is selected from a group consisting of respectively labelled HCV core antigen, HCV NS3 antigen, HCV NS4 antigen and HCV NS5 antigen.

7. The kit of claim 6, wherein the at least one antigen coated on the solid phase support comprises HCV core antigen, HCV NS3 antigen, HCV NS4 antigen and HCV NS5 antigen, which are coated on the solid phase support respectively; the at least one labelled antigen comprises respectively labelled HCV core antigen and HCV NS3 antigen.

8. The kit of claim 6, wherein the at least one antigen coated on the solid phase support comprises HCV core antigen, HCV NS3 antigen, HCV NS4 antigen and HCV NS5 antigen, which are coated on the solid phase support respectively; the at least one labelled antigen comprises respectively labelled HCV core antigen, HCV NS3 antigen, HCV NS4 antigen and HCV NS5 antigen.

9. The kit of claim 5, wherein the kit is used for detection of an antibody produced in a subject infected by *Treponema pallidum*; the at least one antigen coated on the solid phase support is selected from a group consisting of TP15 antigen, TP17 antigen, TP47 antigen and TP45 antigen, which are coated on the solid phase support respectively; the at least one labelled antigen is selected from a group consisting of respectively labelled TP15 antigen, TP17 antigen, TP47 antigen and TP45 antigen.

10. The kit of claim 9, wherein the at least one antigen coated on the solid phase support comprises TP15 antigen, TP17 antigen and TP47 antigen, which are coated on the solid phase support respectively; the at least one labelled antigen comprises respectively labelled TP15 antigen, TP17 antigen and TP47 antigen.

11. The kit of any one of claims 1 to 10, wherein the antigen is present in a form of a polymer, an antigen fragment or a peptide.

12. The kit of any one of claims 1 to 11, wherein an enzyme is used for labelling the antigen or antibody of the second reagent, and the kit further comprises a reaction substrate for the enzyme, preferably, the reaction substrate is 3-(2-spiroadamantane)-4-methoxy-4-(3-phosphoryloxy)-phenyl-1,2-dioxetane.

13. A method for detecting an antibody produced after infection of a pathogen or pathogens in a sample, comprising the following steps:
mixing and reacting the sample with a first reagent and a second reagent,
washing a solution obtained after reaction, and
acquiring a signal value and obtaining a detection result of the antibody,
wherein the first reagent comprises at least one antigen and an anti-human IgM antibody, the antigen and the anti-human IgM antibody are coated on a solid phase support, and
the second reagent comprises at least one labelled antigen and a labelled anti-human IgG antibody,
wherein the at least one antigen coated on the solid phase support and the at least one labelled antigen are capable of binding to a same IgG antibody or a same IgM antibody in the sample.

14. A method for detecting an antibody produced after infection of a pathogen or pathogens in a sample, comprising the following steps:
mixing and reacting the sample with a first reagent and a second reagent,
washing a solution obtained after reaction, and
acquiring a signal value and obtaining a detection result of the antibody,
wherein the first reagent comprises at least one antigen and an anti-human IgG antibody, the antigen and the anti-human IgG antibody are coated on a solid phase support, and
the second reagent comprises at least one labelled antigen and a labelled anti-human IgM antibody,
wherein the at least one antigen coated on the solid phase support and at least one labelled antigen are capable of binding to a same IgG antibody or a same IgM antibody in the sample.

15. The method of claim 13 or 14, wherein the sample is mixed with the first reagent and incubated for a predetermined period of time, and then the second reagent is added and incubated for another period of time.

16. The method of any one of claims 13 to 15, wherein a third reagent is added together with the first reagent and/or the second reagent, and the third reagent contains a blocking agent, preferably, the blocking agent contains one or more components selected from the group consisting of: skimmed milk powder, BSA, gelatin, serum, casein, ovalbumin, animal IgG and surfactant.

17. The method of any one of claims 13 to 16, wherein a fourth reagent is added together with the first reagent, and the fourth reagent contains a reducing agent, preferably, the reducing agent contains one or more components selected from the group consisting of: DTT and β-mercaptoethanol.

18. The method of any one of claims 13 to 17, wherein the pathogen is a virus or a spirochete; preferably, the virus is HIV or HCV, preferably, the spirochete is *Treponema pallidum.*

19. The method of claim 18, wherein the pathogen is HCV; the at least one antigen coated on a solid phase support comprises at least one of HCV core antigen, HCV NS3 antigen, HCV NS4 antigen and HCV NS5 antigen coated on a solid phase support respectively; the at least one labelled antigen comprises at least one of HCV core antigen, HCV NS3 antigen, HCV NS4 antigen and HCV NS5 antigen labelled respectively.

20. The method of claim 19, wherein the at least one antigen coated on a solid phase support comprises HCV core antigen, HCV NS3 antigen, HCV NS4 antigen and HCV NS5 antigen coated on a solid phase support respectively; the at least one labelled antigen comprises HCV core antigen and HCV NS3 antigen labelled respectively.

21. The method of claim 19, wherein the at least one antigen coated on a solid phase support comprises HCV core antigen, HCV NS3 antigen, HCV NS4 antigen and HCV NS5 antigen coated on a solid phase support respectively; the at least one labelled antigen comprises HCV core antigen, HCV NS3 antigen, HCV NS4 antigen and HCV NS5 antigen labelled respectively.

22. The method of claim 18, wherein the pathogen is *Treponema pallidum*; the at least one antigen coated on a solid phase support comprises at least one of TP15 antigen, TP17 antigen, TP47 antigen and TP45 antigen coated on a solid phase support respectively; the at least one labelled antigen comprises at least one of TP15 antigen, TP17 antigen, TP47 antigen and TP45 antigen labelled respectively.

23. The method of claim 22, wherein the at least one antigen coated on a solid phase support comprises TP15 antigen, TP17 antigen and TP47 antigen coated on a solid phase support respectively; the at least one labelled antigen comprises TP15 antigen, TP17 antigen and TP47 antigen labelled respectively.

24. The method of any one of claims 13 to 23, wherein the antigen is present in the form of a polymer, an antigen fragment or a peptide.

25. The method of any one of claims 13 to 24, wherein the sample is selected from whole blood, plasma and serum.
